(19) Europäisches Patentamt
European Patent Office
Office européen des brevets

(11) **EP 2 670 454 B1**

(12) **EUROPÄISCHE PATENTSCHRIFT**

(45) Veröffentlichungstag und Bekanntmachung des Hinweises auf die Patenterteilung:
**16.12.2015 Patentblatt 2015/51**

(21) Anmeldenummer: **12702970.0**

(22) Anmeldetag: **01.02.2012**

(51) Int Cl.:
***A61M 1/36*** *(2006.01)*

(86) Internationale Anmeldenummer:
**PCT/EP2012/000438**

(87) Internationale Veröffentlichungsnummer:
**WO 2012/104072 (09.08.2012 Gazette 2012/32)**

(54) **VERFAHREN ZUM STEUERN EINER BLUTBEHANDLUNGSVORRICHTUNG, STEUEREINRICHTUNG, ZUGABEEINRICHTUNG UND BLUTBEHANDLUNGSVORRICHTUNG**

METHOD FOR CONTROLLING A BLOOD TREATING DEVICE, CONTROL DEVICE, FEED DEVICE, AND BLOOD TREATING DEVICE

PROCÉDÉ DE COMMANDE D'UN DISPOSITIF DE TRAITEMENT DU SANG, SYSTÈME DE COMMANDE, SYSTÈME DE DISTRIBUTION ET DISPOSITIF DE TRAITEMENT DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.02.2011 DE 102011010406**
**04.02.2011 US 201161439387 P**

(43) Veröffentlichungstag der Anmeldung:
**11.12.2013 Patentblatt 2013/50**

(73) Patentinhaber: **Fresenius Medical Care Deutschland GmbH**
**61352 Bad Homburg (DE)**

(72) Erfinder: **POHLMEIER, Robert**
**61350 Bad Homburg (DE)**

(74) Vertreter: **Bobbert & Partner**
**Patentanwälte PartmbB**
**Postfach 1252**
**85422 Erding (DE)**

(56) Entgegenhaltungen:
**WO-A2-2010/029401     US-A1- 2005 085 760**
**US-A1- 2007 066 928**

EP 2 670 454 B1

**Beschreibung**

Die vorliegende Erfindung betrifft ein Verfahren zum Steuern oder Regeln einer Vorrichtung zum extrakorporalen Behandeln von Blut gemäß Anspruch 1. Sie betrifft ferner eine Steuereinrichtung gemäß Anspruch 9, eine Benutzeroberfläche gemäß Anspruch 10, eine Einrichtung zum Zugeben einer Calciumlösung in den extrakorporalen Blutkreislauf gemäß Anspruch 12, sowie eine Vorrichtung zum extrakorporalen Behandeln von Blut gemäß Anspruch 13.

Bei bestimmten extrakorporalen Blutbehandlungen erfolgt zum Verhindern einer Koagulation des extrakorporal geführten Bluts eine Koagulationsprophylaxe. Bei einem bekannten diesem Zweck dienenden Verfahren wird eine Koagulationsneigung des extrakorporal strömenden Bluts gesenkt, indem dem Blut zunächst Citrat zugegeben wird. Durch die Zugabe des Citrats wird Calcium vermehrt permeabel durch die Membran des eingesetzten Filters hindurch übertragen. Dadurch und durch die Verwendung während der Blutbehandlung auch von Lösungen, welche kein Calcium aufweisen, kommt es zu einem Calciumverlust aus dem Blut. Nach erfolgtem Behandeln des Bluts wird dem Blut Calcium in Form einer calciumhaltigen Lösung zugegeben, bevor es in den Körper des Patienten rückgeführt wird. Aus der Praxis sind Verfahren und Vorrichtungen zum Bestimmen einer zum Erzielen dieser Wirkung erforderlichen Menge oder Konzentration an Calcium bekannt.

Aus der US 2007/0066928 A1 ist ein System zum Optimieren eine Zitrat-Antikoagulation bekannt. Aus der WO 2010/029401 A2 ist eine Blutbehandlungsvorrichtung bekannt. Aus der US 2005/0085760 A1 sind Vorrichtungen zum Synchronisieren von Pumpen bekannt.

Eine Aufgabe der vorliegenden Erfindung ist es, ein weiteres Verfahren zum Steuern oder Regeln einer Vorrichtung zum extrakorporalen Behandeln von Blut unter Zugabe von Zitrat bzw. Citrat (im Folgenden auch mit Ci abgekürzt) und Kalzium bzw. Calcium (im Folgenden auch mit Ca abgekürzt) vorzuschlagen. Zudem sollen eine geeignete Steuereinrichtung, eine entsprechende Benutzeroberfläche sowie Einrichtungen und Vorrichtungen angegeben werden.

Die erfindungsgemäße Aufgabe wird durch ein Verfahren mit den Merkmalen des Anspruchs 1 gelöst. Sie wird ferner durch eine Steuereinrichtung mit den Merkmalen gemäß Anspruch 9, eine Benutzeroberfläche mit den Merkmalen gemäß Anspruch 10, eine Einrichtung mit den Merkmalen gemäß Anspruch 12 und durch eine Vorrichtung mit den Merkmalen gemäß Anspruch 13 gelöst.

Gemäß der vorliegenden Erfindung wird ein Verfahren zum Steuern oder Regeln einer Vorrichtung vorgeschlagen, welche zum extrakorporalen Behandeln von Blut dient, wobei das Blut in einem extrakorporalen Blutkreislauf unter Zugabe von Citrat zur Antikoagulation oder Koagulationsprohylaxe behandelt wird. Die geregelte oder gesteuerte Vorrichtung weist außerdem eine Zugabeeinrichtung zum Zugeben einer Calciumlösung in den extrakorporalen Blutkreislauf auf. Alternativ dient das erfindungsgemäße Verfahren dem Steuern oder Regeln einer solchen Zugabeeinrichtung.

Das erfindungsgemäße Verfahren umfasst ein Ermitteln und/oder Ausgeben eines Signals an die Zugabeeinrichtung zum Verändern einer Einstellung der Zugabeeinrichtung, mittels welcher dem extrakorporal strömenden Blut Calcium zugegeben wird. Dabei entspricht die Einstellung einer Abgabe von Calcium, einer Calciumdosierung oder -konzentration oder -menge oder -rate oder bewirkt eine solche.

Das erfindungsgemäße Verfahren umfasst ferner ein Festlegen des Signals unter Berücksichtigung eines Calciumgehalts oder einer Calciumkonzentration einer beim oder zum extrakorporalen Behandeln des Bluts verwendeten Substituatlösung.

Ferner umfasst das erfindungsgemäße Verfahren ein Ermitteln des Wertebereichs eines zulässigen oder zugelassenen Einstellbereichs (oder Bereichs für die Einstellung) für das mittels der Zugabeeinrichtung in den extrakorporalen Blutkreislauf eingebrachte oder einzubringende Calcium, insbesondere dessen Menge, Konzentration oder Dosierung.

Die erfindungsgemäße Steuereinrichtung ist vorgesehen und/oder programmiert zum Ausführen des erfindungsgemäßen Verfahrens.

Die erfindungsgemäße Benutzeroberfläche weist eine erfindungsgemäße Steuereinrichtung auf und/oder ist funktionell mit einer solchen verbunden. Die erfindungsgemäße Benutzeroberfläche weist eine Eingabeeinrichtung auf. Die Eingabeeinrichtung ist vorgesehen zum Angeben bzw. Eingeben (im Sinne eines Mitteilens) einer Calciumdosierung oder - menge oder -konzentration durch den Anwender oder den Benutzer der Benutzeroberfläche, deren Zugabe mittels der Steuereinrichtung eingeleitet oder bewirkt werden soll.

Die Eingabeeinrichtung kann eine Taste oder eine Tastatur, ein Schieber, ein Touchscreen oder ein anderweitig zur Eingabe von Daten vorbereitetes Display oder Mittel, eine Computermaus, oder dergleichen sein.

Die erfindungsgemäße Einrichtung zum Zugeben einer Calciumlösung in den extrakorporalen Blutkreislauf ist programmiert zum Durchführen, insbesondere zum automatisierten Durchführen, des erfindungsgemäßen Verfahrens und/oder wird entsprechend angesteuert mittels der erfindungsgemäßen Steuereinrichtung.

Die erfindungsgemäße Vorrichtung ist vorgesehen zum extrakorporalen Behandeln von Blut. Sie ist zudem zum Ausführen oder Durchführen des erfindungsgemäßen Verfahrens ausgestaltet und vorgesehen, und/oder weist wenigstens eine erfindungsgemäße Steuereinrichtung, und/oder eine erfindungsgemäße Einrichtung zum Zugeben einer Calciumlösung in den extrakorporalen Blutkreislauf und/oder eine erfindungsgemäße Benutzeroberfläche auf.

Alle mit dem erfindungsgemäßen Verfahren erzielbaren Vorteile lassen sich in bestimmten erfindungsgemäßen Ausführungsformen ungeschmälert mit jedem der erfindungsgemäßen Gegenstände erzielen.

Ein digitales Speichermedium, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein Computerprogramm-Produkt weist einen auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf.

Ein maschinenlesbarer Träger bezeichnet in bestimmten Ausführungsformen der vorliegenden Erfindung einen Träger, der von Software und/oder Hardware interpretierbare Daten oder Informationen enthält. Der Träger kann eine Diskette, eine CD, DVD, ein USB-Stick, eine Flashcard, eine SD-Karte und dergleichen sein.

Ein Computerprogramm weist einen Programmcode auf zur Veranlassung der maschinellen Schritte eines erfindungsgemäßen Verfahrens, wenn das Computerprogramm auf einem Computer abläuft.

Auch für das Computerprogramm-Produkt und das Computerprogramm gilt, dass alle, einige oder manche der maschinell durchgeführten Schritte des erfindungsgemäßen Verfahrens veranlasst werden.

Erfindungsgemäße Ausführungsformen können eines oder mehrere der im Folgenden genannten Merkmale aufweisen. Erfindungsgemäße Ausführungsformen sind zudem Gegenstand der abhängigen Ansprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

In bestimmten erfindungsgemäßen Ausführungsformen ist die Zugabeeinrichtung zum Zugeben einer Calciumlösung in den extrakorporalen Blutkreislauf eine Pumpe, insbesondere eine Calciumpumpe.

In bestimmten erfindungsgemäßen Ausführungsformen ist die Zugabeeinrichtung zum Zugeben einer Calciumlösung ausschließlich zum Zugeben von Calciumlösung zum Erzielen eines Calciumausgleichs oder einer Calciumkompensation vorgesehen.

Das - in manchen erfindungsgemäßen Ausführungsformen automatisch oder mittels entsprechender Vorrichtungen durchgeführte - Ermitteln des Wertebereichs eines zulässigen oder zugelassenen Einstellbereichs für das mittels der Zugabeeinrichtung in den extrakorporalen Blutkreislauf eingebrachte oder einzubringende Calcium, insbesondere dessen Menge, Konzentration oder Dosierung, erfolgt in bestimmten erfindungsgemäßen Ausführungsformen bei jeder Einstellung oder Veränderung erneut. In manchen erfindungsgemäßen Ausführungsformen erfolgt das Ermitteln mehrfach während einer Blutbehandlung, soweit erforderlich. Der Wertebereich oder dessen niedrigster Wert werden in einigen erfindungsgemäßen Ausführungsformen somit dynamisch ermittelt.

In manchen erfindungsgemäßen Ausführungsformen ist das Ermitteln des Wertebereichs eines zulässigen oder zugelassenen Einstellbereichs oder Bereichs für die Einstellung ein Festlegen eines Wertebereichs eines zulässigen oder zugelassenen Einstellbereichs oder Bereichs für die Einstellung, insbesondere eines dem Benutzer oder Einsteller - beispielsweise in einer Benutzeroberfläche oder mittels einer Schalterbelegung - angebotenen oder ausschließlich angebotenen Einstellbereichs oder Bereichs für die Einstellung. In diesen Ausführungsformen der vorliegenden Erfindung ist ein Einstellen von Werten, die außerhalb des Einstellbereichs liegen, durch den Benutzer ausgeschlossen. Alternativ zu einem Ausschließen dieser Werte oder ergänzend hierzu kann die Ausgabe einer Anzeige oder eines Alarms erfolgen, wenn solche Werte eingestellt werden sollten.

Das Ermitteln des Wertebereichs eines zulässigen oder zugelassenen Einstellbereichs erfolgt automatisch, ohne Zutun des Benutzers.

In manchen erfindungsgemäßen Ausführungsformen erfolgt das Ermitteln des Wertebereichs nicht vor oder erst nach Beginn der Behandlung, in gewissen erfindungsgemäßen Ausführungsformen erfolgt das Ermitteln nicht vor oder erst nach dem Einlegen oder Verwenden einer Quelle für Substituatlösung.

In bestimmten erfindungsgemäßen Ausführungsformen erfolgt das Ermitteln des Wertebereichs unter Einbezug, unter mathematischer Berücksichtigung, unter Berücksichtigung von oder basierend auf einem Calciumgehalt oder einer Calciumkonzentration einer beim oder zum extrakorporalen Behandeln des Bluts verwendeten Substituatlösung.

In einigen erfindungsgemäßen Ausführungsformen sind geeignete, optional entsprechend konfigurierte oder programmierte Vorrichtungen vorgesehen, um einige, manche, beliebige oder alle der Schritte oder Teilschritte des erfindungsgemäßen Verfahrens auszuführen.

In einigen erfindungsgemäßen Ausführungsformen ist unter dem "Steuern" ein "Regeln" zu verstehen. Beide Begriffe sind, wo immer für den Fachmann als ausführbar erkennbar, gleichermaßen von der vorliegenden Erfindung umfasst. Wenn daher von einer Steuereinrichtung oder einem Steuern die Rede ist, kann auch eine Regeleinrichtung oder ein Regeln zu verstehen sein, sofern technisch sinnvoll.

In bestimmten erfindungsgemäßen Ausführungsformen umfasst das Verfahren ein Festsetzen eines als kleinstzulässig erachteten unteren Wertes - im Folgenden zum Teil auch als "unterer Schwellenwert" oder als "minimale Calciumdosis" bezeichnet und daher als Synonyme zu verstehen wann immer dies dem Fachmann als sinnvoll erscheint - des Einstellbereichs, insbesondere auf einen Wert gleich oder größer Null.

In manchen erfindungsgemäßen Ausführungsformen umfasst das Verfahren das - insbesondere automatisch ablaufende - Erkennen eines an der Vorrichtung zum extrakorporalen Behandeln von Blut eingestellten oder ablaufenden Blutbehandlungsmodus. Wird dabei erkannt, dass ein Blutbehandlungsmodus eingestellt ist oder abläuft, bei welchem eine Substitution mittels Substituat erfolgt, so kann vorteilhaft automatisch eine Berechnung des erforderlichen Calciumausgleichs mittels des erfindungsgemäßen Verfahrens erfolgen. Insbesondere kann hierbei ein Calciumgehalt des verwendeten Substituats vorteilhaft automatisch mitberücksichtigt werden.

In einigen erfindungsgemäßen Ausführungsformen umfasst das Verfahren ein Ermitteln des als kleinstzulässig erachteten unteren Schwellenwerts des Wertebereichs des Einstellbereichs. Da bei Verwenden einer calciumhaltigen Substitutionslösung bzw. Substituatslösung bereits mittels des Substituats eine Calcium-Substitution oder -gabe erfolgt, beträgt eine Calciumdosierung aufgrund der mittels der Substitutionslösung applizierten Calciummenge bereits mehr als 0,0 mmol/l. Nimmt man eine Schrittweite von 0,1 mmol/l an, so beträgt die minimale Calciumdosis bereits 0,1 mmol/l.

In manchen erfindungsgemäßen Ausführungsformen umfasst das Verfahren ein Ermitteln des als kleinstzulässig - bzw. des kleinsten als zulässig - erachteten unteren Schwellenwerts des Wertebereichs des Einstellbereichs nach

Formel (1):

$$Ca\_Dosis\_\min = \frac{[Ca]\_Sub * Q\_Sub + [Ca]\_Lsg * Q\_Ca\_\min}{Q\_Fil + Q\_Ca\_\min}$$

In Formel (1) gilt:

Ca_Dosis_min ist die minimale Calciumdosis bzw. der als kleinstzulässig erachtete Wert oder untere Schwellenwert des Wertebereichs; [Ca]_Sub ist die Calciumkonzentration des Substituats; Q_Sub ist der Substituatfluss; [Ca]_Lsg ist die Calciumkonzentration der Calciumlösung; Q_Ca_min ist der minimale Calciumfluss; und Q_Fil ist der Filtratfluss. Allein zu einfacheren Darstellung von Formel (1) ist der Filtratfluss Q_Fil hier definiert als der Fluss des Filtrats aus dem Filter heraus, der um den Teil des Filtrats vermindert ist, der das mit dem Calciumfluss (Q_Ca) infundierte Volumen kompensiert. Tatsächlich strömt durch die Filtratleitung dabei zusätzlich zum hier definierten Filtratfluss auch der Fluss, der das mit dem Calciumfluss (Q_Ca) infundierte Volumen kompensiert.

Der so definierte Filtratfluss (Q_Fil) entspricht dabei in bestimmten erfindungsgemäßen Ausführungsformen der Summe von Substituatfluss (Q_Sub), Dialysatfluss (Q_Dia), Citratfluss (Q_Ci), Nettofluss der Ultrafiltration (Q_UF), und Heparinfluss (Q_Hep). Jedoch kann der Filtratfluss in anderen erfindungsgemäßen Ausführungsformen einer Summe von zwei oder mehr der im vorausgehenden Satz genannten Flüssen, in beliebigen Kombinationen, entsprechen. In manchen erfindungsgemäßen Ausführungsformen sind die Flüsse, welche aufsummiert den Filtratfluss ergeben, nicht auf die hier genannten Flüsse beschränkt. So kann bei Ausgestaltung einer Behandlungsvorrichtung mit beispielsweise einer Phosphatpumpe auch ein Phosphatfluss oder ein entsprechender Kompensationsstrom in die Summe eingehen, welche den Filtratfluss bildet.

Der Filtratfluss ist in manchen erfindungsgemäßen Ausführungsformen der Fluss jener Flüssigkeit, welche in der Behandlungsvorrichtung gebildet wird. Dieser Fluss wird ggf. um den Anteil vermindert, der das mit dem Calciumfluss infundierte Volumen kompensiert.

Die zuvor genannten Flüsse - und auch ein Calciumfluss - können jeweils berechnete, an den entsprechenden Pumpen eingestellte oder ermittelte Flüsse oder Raten sein.

Wenn der minimal mögliche Calciumfluss (Q_Ca) einem Stopp bzw. völligen Anhalten des Calciumflusses entspricht, so gilt Q_Ca = 0. Formel (1) kann daher in den entsprechenden erfindungsgemäßen Ausführungsformen auch als Formel (1'):

$$Ca\_Dosis\_\min = \frac{[Ca]\_Sub * Q\_Sub}{Q\_Fil}$$

verwendet werden.

Da der Heparinfluss (Q_Hep) oftmals sehr gering ist, kann Q_Hep in bestimmten erfindungsgemäßen Ausführungsformen vernachlässigt oder mit Null bestimmt werden und trägt dann ebenfalls nicht zum Filtratfluss (Q_Fil) bei bzw. wird nicht hierzu gezählt.

Der mittels der Formel (1) oder (1') errechnete Wert wird in bestimmten erfindungsgemäßen Ausführungsformen auf volle Zehntel mmol/l aufgerundet. Letzteres kann den Implementierungs- und/oder Steuerungsaufwand vorteilhaft und ggf. erheblich senken.

Formel (1) lässt sich herleiten aus der Überlegung, dass bei der Bestimmung der Calciumdosis für einen Calciumausgleich die gesamte Calciumgabe berücksichtigt werden muss, und dass die minimal mögliche Calciumdosis bei einem minimal möglichen Calciumfluss von Null erreicht ist, siehe Formel (2):

$$Ca\_Dosis = \frac{[Ca]\_Sub * Q\_Sub + [Ca]\_Lsg * Q\_Ca}{Q\_Fil + Q\_Ca}$$

Der Nenner von Formel (2) gibt den Fluss des Filtrats aus dem Filter heraus an und ist um den gewünschten Wasserentzug (Q_UF) höher als die Summe aller Flüsse (insbesondere von Substituat, Dialysat, Citrat und Heparin (jeder Fluss und insbesondere ein Heparinfluss wird nur berücksichtigt, sofern die Gabe des entsprechenden Fluids oder Stoffs wie beispielsweise Heparin hier überhaupt erfolgt)) an, die in Richtung zum extrakorporalen Blutkreislauf appliziert werden, einschließlich des Calciumflusses.

Formel (1) geht dabei erkennbar aus Formel (2) für den Fall hervor, dass man den Fluss von Calcium Q_Ca gleich dem minimal möglichen Calciumfluss Q_Ca_min setzt oder entsprechend annimmt. Für diesen Fall erhält man mit Formel (1) einen Grenzwert im Sinne einer minimalen oder minimal möglichen Dosis. Diese erhält man, wenn die Calciumzufuhr aus einer Calciumquelle, welche zum Zweck des Calciumausgleichs erfolgt, minimiert wird.

Ein minimierter Calciumfluss oder eine minimierte Calciumzufuhr ist vorliegend zu verstehen als jener Fluss, der mittels einer Calciumpumpe dann erzeugt wird, wenn die Calciumpumpe auf die in ihrem Normalgebrauch niedrigst mögliche Fördereinstellung oder Förderleistung eingestellt ist. Bei dieser Einstellung, bei welcher die Pumpe nicht völlig abgestellt ist und der von ihr erzeugte Fluss folglich nicht Null ist, wird im Zusammenhang mit der vorliegenden Erfindung der resultierende Calciumfluss auch als der "minimal möglichen" Calciumfluss Q_Ca_min bezeichnet.

Aus den o. g. Formeln ergibt sich folgende Formel (3) für den Fluss Q_Ca der Calciumlösung, welche üblicherweise die Dimension [ml/h] hat:

$$Q\_Ca = \frac{Ca\_Dosis * Q\_Fil - [Ca]\_Sub * Q\_Sub}{[Ca]\_Lsg - Ca\_Dosis}$$

Der Calciumfluss Q_Ca kann erfindungsgemäß zum Ansteuern oder Regeln der Calciumpumpe verwendet werden, wie dies in bestimmten erfindungsgemäßen Ausführungsformen vorgesehen ist.

Unter einer Calciumdosis bzw. Ca-Dosis wird eine Menge an Calcium bezogen auf ein Flüssigkeitsvolumen verstanden. In manchen erfindungsgemäßen Ausführungsformen wird unter einer Calciumdosis bzw. Ca-Dosis das Verhältnis von Calcium (in Millimol [mmol]) zum Filtrat oder auch Effluent bzw. Abfluss (in Liter [l]) verstanden. Dabei werden die Begriffe Filtrat, Effluent und Abfluss vorliegend synonym verwendet. Gemeint ist dabei jeweils die Mischung aus gebrauchtem Dialysat und durch die Membran des Filters hindurch filtrierter Flüssigkeit, die bei oder nach der Behandlung verworfen wird.

Unter dem Filtrat wird in einigen erfindungsgemäßen Ausführungsformen die Summe aller bei oder nach Behandlung verworfenen Flüssigkeiten verstanden (z. B. das gesamte Effluent). In manchen erfindungsgemäßen Ausführungsformen wird der Calciumfluss oder ein diesem entsprechender Kompensationsfluss ausdrücklich nicht hinzugezählt, in anderen hingegen schon. In bestimmten erfindungsgemäßen Ausführungsformen wird der Heparinfluss oder ein diesem entsprechender Kompensationsfluss ausdrücklich nicht hinzugezählt, in anderen hingegen schon.

Unter einer Calciumdosis wird in einigen erfindungsgemäßen Ausführungsformen die insgesamt in den extrakorporalen Blutkreislauf infundierte Calciummenge (also die Summe des Calciums aus der Substitutionslösung und des mittels der Zugabeeinrichtung zum Zwecke des Calciumausgleichs zugegebenen Calcium aus der Calciumlösung) in Relation zur gebildeten Menge an Filtrat (oder dem von der Filtratpumpe geförderten Volumen) verstanden.

In manchen erfindungsgemäßen Ausführungsformen erlaubt das Verfahren ein Einstellen der Calciumdosierung, -menge oder - konzentration durch den Benutzer bzw. Anwender der Vorrichtung oder der Zugabeeinrichtung explizit nur im Wertebereich des Einstellbereichs und/oder nicht unterhalb des als kleinstzulässig erachteten unteren Wertes oder des Schwellenwertes.

Dies kann sichergestellt sein dadurch, dass andere Werte als die zugelassenen nicht angezeigt und damit nicht auswählbar gemacht werden. Sichergestellt werden kann dies ferner dadurch, dass Hinweise, Alarme oder Fehlermeldungen ausgegeben werden, sollte ein nicht zulässiger Wert vom Benutzer bzw. Anwender eingegeben werden oder eingegeben werden wollen.

Auf diese Weise kann eine schädliche, bewusst oder unbewusst erfolgende Einstellung durch den Anwender vorteilhaft vermieden werden. Ebenso kann es möglich sein, hierdurch vermeidbare Alarme zu verhindern.

In einigen Ausführungsformen umfasst das erfindungsgemäße Verfahren ein Ausgeben eines Hinweises bei erfolgtem oder erfolgendem Wechsel eines Behältnisses für das verwendete Substituat oder bei erfolgter, erkannter oder geplanter Veränderung der Zusammensetzung oder des bzw. der Calciumgehalts oder -konzentration des verwendeten Substituats.

Auf diese Weise kann vorteilhaft sichergestellt werden, dass einer zwischen verschiedenen Produkten von Substitutionslösungen - welche ggf. auch noch von verschiedenen Herstellern bezogen werden - variierenden Calciumkonzentration zuverlässig Rechnung getragen wird. Der Hinweis, welcher bei erfolgtem oder erfolgendem Wechsel eines Behältnisses für das verwendete Substituat in bestimmten Ausführungsformen erfolgt, ermöglicht eine manuelle oder - nach Eingabe bestimmter Angaben zur von nun an verwendeten Substitutionslösung - automatische Anpassung der mittels der Zugabeeinrichtung zugegebenen Calciumdosis.

Ein in bestimmten erfindungsgemäßen Ausführungsformen vorgesehener Einstellbereich für die Calciumkonzentration der verwendeten Substitutionslösung umfasst Werte zwischen 1,00 mmol/l und 2,00 mmol/l. Andere Wertebereiche sind allerdings ebenfalls von der Erfindung umfasst. Eine in manchen Ausführungsformen vorgesehene Schrittweite für den Einstellbereich beträgt 0,25 mmol/l. Eine Default- oder Grundeinstellung für die Substitutionslösung kann 1,5 mmol/l betragen, falls vorgesehen. Jeder Defaultwert kann vom Anwender und/oder automatisch veränderbar sein.

In manchen erfindungsgemäßen Ausführungsformen umfasst das Verfahren das Ausgeben eines Hinweises, welcher im Zuge eines Wechsels oder Ersetzens des Substituatbeutels oder im Zusammenhang hiermit erscheint und dem Anwender signalisiert, welche Calciumkonzentration die zu verwendende Substituatlösung haben muss. Der Hinweis kann auf einem Monitor angezeigt werden. Der Hinweis kann eingebunden sein in einen Hinweis darauf, dass ein Wechsel des Substituatbeutels zu erfolgen hat. Ein derartiger Hinweis kann vorteilhaft zur Behandlungssicherheit beitragen.

In gewissen erfindungsgemäßen Ausführungsformen umfasst das Verfahren ein Anpassen des als kleinstzulässig erachteten Wertes des Wertebereichs oder unteren Schwellenwerts aufgrund von Veränderung der Förderleistung der Blutpumpe und/oder der Dialysatpumpe oder aufgrund von Veränderung der aus deren Tätigkeiten resultierenden Flüssen.

Das Anpassen des als kleinstzulässig erachteten unteren Wertes oder Schwellenwerts erfolgt in bestimmten erfindungsgemäßen Ausführungsformen vorteilhaft automatisch, also steuerungsseitig oder maschinenseitig, und ohne Zutun des Benutzers. Auf diese Weise kann eine an sich nicht erforderliche Intervention des Anwenders vorteilhaft unterbleiben mit den hiermit bekannten Vorteilen.

Das Anpassen des als kleinstzulässig erachteten unteren Wertes oder Schwellenwerts ist in bestimmten erfindungsgemäßen Ausführungsformen in einigen Fällen ein Anheben des Wertes oder Schwellenwerts.

In manchen erfindungsgemäßen Ausführungsformen umfasst das Verfahren ein - dauerhaftes oder vorübergehendes - Reduzieren der Rate oder des Flusses, mit welcher dem extrakorporalen Blutkreislauf Substituat bzw. Substituatlösung zugeführt wird. Bei der Reduzierung oder beim Ermitteln, wie sehr oder in welchem Maß reduziert wird, wird eine erfolgte, erwartete oder geplante - dauerhafte oder vorübergehende - Reduzierung des innerhalb des extrakorporalen Blutkreislaufs herrschenden Blutflusses berücksichtigt, beispielsweise auf mathematische Weise. Hierbei kann vorteilhaft dazu beigetragen werden, dass eine übermäßige Hämokonzentration des Blutes am Ausgang des Filters vermieden wird. Immerhin kann eine hohe Hämokonzentration das Risiko einer Gerinnung im extrakorporalen Blutkreislauf erhöhen.

In einigen erfindungsgemäßen Ausführungsformen umfasst das Verfahren ein Zulassen eines Absenkens der mittels der Zugabeeinrichtung zugegebenen Calciumdosis unter den zuvor als kleinstzulässig erachteten unteren Wert oder unter den zuvor als unteren Schwellenwert des Einstellbereichs erachteten Wert durch den Benutzer, für den Fall dass - oder wenn - nach Änderung des Blutflusses die sich aufgrund der Änderung ergebende minimale Calciumdosis niedriger liegt als vor der Änderung. Dies ermöglicht es vorteilhaft, eine zuvor noch als notwendig erkannte Einschränkung wieder zu lockern oder aufzugeben. Damit steht es dem Anwender - oder der Maschine bei automatischer Anpassung - anschließend frei, diese nun freigegebene oder zulässige Einstellmöglichkeit (erstmalig oder ggf. erneut) zu nutzen.

Ein zuvor als kleinstzulässig erachteter unterer Wert oder unterer Schwellenwert ist in bestimmten erfindungsgemäßen Ausführungsformen jeweils ein Wert, welcher bei einer vorhergehenden Berechnung oder Bestimmung gemäß dem erfindungsgemäßen Verfahren als solcher festgesetzt, errechnet, eingestellt oder ermittelt wurde. In einigen erfindungsgemäßen Ausführungsformen ist der Wert "zuvor" ein vorangegangener oder der zuletzt erfindungsgemäß festgesetzte, errechnete, eingestellte oder ermittelte Wert oder Schwellenwert.

In manchen dieser Ausführungsformen kann vorgesehen sein, einen entsprechenden Hinweis an den Benutzer auszugeben.

Ein Hinweis, wie hierin an verschiedenen Stellen verwendet, kann in bestimmten erfindungsgemäßen Ausführungsformen ein optischer oder akustischer Alarm, ein Text in einem Displayfenster oder dergleichen sowie Kombinationen hieraus sein.

In bestimmten erfindungsgemäßen Ausführungsformen weist die Benutzeroberfläche eine Anzeige auf, welche vorgesehen ist und/oder angesteuert wird, um den als kleinstzulässig erachteten unteren Wert oder den unteren Schwellenwert anzuzeigen.

In bestimmten erfindungsgemäßen Ausführungsformen ist es vorgesehen, ergänzend oder alternativ zu dem als kleinstzulässig erachteten unteren Wert oder dem unteren Schwellenwert oder der minimalen Calciumdosis einen als höchstzulässig erachteten oberen Wert oder einen oberen Schwellenwert oder die maximale Calciumdosis anzugeben. Was hierin zu dem als kleinstzulässig erachteten unteren Wert oder dem unteren Schwellenwert oder der minimalen Calciumdosis ausgeführt ist, trifft erfindungsgemäß auch für den als höchstzulässig erachteten oberen Wert oder den oberen Schwellenwert oder die maximale Calciumdosis zu und fällt unter die vorliegende Erfindung, soweit dies für den Fachmann als sinnvoll und/oder ausführbar erkennbar ist.

In einigen erfindungsgemäßen Ausführungsformen ist die Vorrichtung ausgestaltet als Dialysevorrichtung, insbesondere als Hämodiafiltrationsvorrichtung oder Hämofiltrationsvorrichtung.

In bestimmten erfindungsgemäßen Ausführungsformen ist die Vorrichtung ausgestaltet als Dialysevorrichtung, welche zur Blutbehandlung sowohl mittels Hämodiafiltration als auch mittels Hämofiltration und ggf. weiteren Behandlungsoptionen ausgestaltet und vorgesehen ist.

So kann z. B. anstatt einer maschinenseitig bereits implementierten Hämofiltration unter Ci-Ca-Antikoagulation (z. B. CVVHD: Kontinuierliche veno-venöse Hämodialyse) mit einer erfindungsgemäßen Vorrichtung wahlweise auch eine Hämodiafiltration unter Ci-Ca-Antikoagulation (z. B. CVVHDF: Kontinuierliche veno-venöse Hämodiafiltration) durchgeführt werden. Die erforderlichen Anpassungen der Vorrichtung an das eine oder das andere der beiden Verfahren können dabei insbesondere bei Verwenden einer erfindungsgemäßen Benutzeroberfläche derart gering gehalten sein, dass eine Auswahl des einen Verfahrens gemessen an der Auswahl des anderen Verfahrens keine nennenswerte Umstellung oder Umgewöhnung des Anwenders bedeutet.

In bestimmten erfindungsgemäßen Ausführungsformen wird die Hämodiafiltration unter Ci-Ca-Antikoagulation (z. B. CVVHDF) mit einer Substitution (z. B. mittels einer "multiBic" Lösung aus dem Hause der Anmelderin) in Postdilution durchgeführt. Die entsprechenden Voraussetzungen an Steuerungen, Leitungen usw. sind dabei gewährleistet bzw. vorgesehen.

Bestimmte erfindungsgemäße Ausführungsformen weisen neben den an anderer Stelle bereits genannten Vorteile einen oder mehrere der im Folgenden genannten Vorteile auf.

Bei Hämodiafiltrationsverfahren wird dem Blut mehr Flüssigkeit entzogen als dies zur Entwässerung klinisch erforderlich ist. Auf diese Weise können dem Blut in höherem Maße urämische Toxine entzogen werden. Die Flüssigkeitsbilanz wird durch Einbringen von Substituat in den extrakorporalen Blutkreislauf kompensiert. Da die verwendete Substitutionslösung Calcium enthält oder enthalten kann, wird das in der Substitutionslösung enthaltene Calcium bei einem Calciumausgleich erfindungsgemäß vorteilhaft mit berücksichtigt.

In bestimmten erfindungsgemäßen Ausführungsformen erlaubt das Verfahren vorteilhaft, den Widerspruch aufzulösen bzw. eine Lösung hierfür anzubieten, dass beim Calciumausgleich auch das in einer Hämofiltration oder in einem Substituat enthaltene Calcium berücksichtigt wird und zudem in bestimmten solcher Konstellationen und Parameterausprägungen der Calciumfluss an sich bzw. basierend auf der verwendeten mathematischen Formel rein rechnerisch negativ eingestellt werden müsste. Die erfindungsgemäße Lösung besteht in diesen Ausführungsformen darin - anders als rechnerisch gefordert, technisch aber nicht sinnvoll - eine technisch sinnvolle und umsetzbare Lösung anzugeben. Diese kann darin bestehen, den Calciumfluss auf einem Mindestwert zu halten.

Die vorliegende Erfindung bietet ferner in einigen Ausführungsformen eine betont nutzerfreundliche Implementierung des erfindungsgemäßen Verfahrens. Dieser Vorteil stützt sich auch darauf, dass der Anwender mit ihm bekannten Benutzeroberflächen der verwendeten Behandlungsvorrichtung arbeiten kann. Die in Frage stehenden Benutzeroberflächen unterscheiden sich nur geringfügig von jenen, die dem Anwender bereits für ein anderes bekanntes Verfahren zur Verfügung stehen. Der Anwender kann weiterhin mit dem ihm bekannten Erscheinungsbild der

Benutzeroberflächen arbeiten.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:

Fig. 1     zeigt schematisch vereinfacht einen Blutkreislauf mit einer Zugabeeinrichtung zum Zugeben eines calciumhaltigen Substituats;

Fig. 2     zeigt mögliche Einstellbereiche für eine Calciumdosierung mit dem Ziel des Calciumausgleichs sowie Grenzen des Einstellbereichs; und

Fig. 3     zeigt eine erfindungsgemäße Benutzeroberfläche zur Durchführung des erfindungsgemäßen Verfahrens.

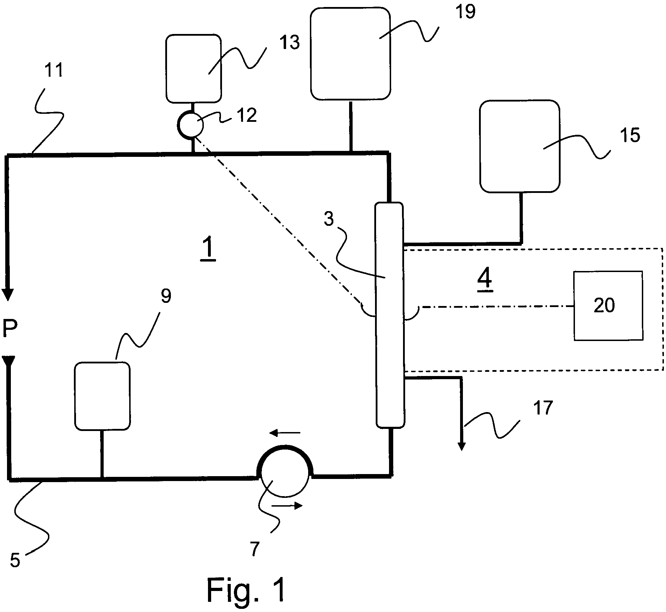

**Fig. 1** zeigt schematisch vereinfacht einen Blutkreislauf 1 mit einem Dialysefilter bzw. Blutfilter oder Filter 3 zum Durchführen einer extrakorporalen Blutbehandlung, bei welcher mittels Citrat-Calcium-Gabe auf die Blutgerinnung eingewirkt wird. Der Filter 3 ist verbunden mit einer erfindungsgemäßen Vorrichtung 4 zum extrakorporalen Behandeln von Blut, welche in Fig. 1 mittels Strichlinien nur angedeutet ist.

Der Blutkreislauf 1 umfasst eine von einem Patienten P wegführende arterielle Patientenleitung bzw. Leitung 5 mit einer Blutpumpe 7. Aus einer Quelle für Citratlösung, hier beispielhaft als ein Citratbeutel 9 ausgestaltet, wird eine Citratlösung in die Leitung 5 abgegeben. Aus der Quelle für Citratlösung wird beispielsweise 4%-iges $Na_3$Citrat zugeführt.

Der Blutkreislauf 1 umfasst ferner eine zum Patienten P hinführende venöse Patientenleitung bzw. Leitung 11. Eine Zugabeeinrichtung, hier als Calciumpumpe 12 ausgestaltet, ist vorgesehen, um aus einer Quelle für Calciumlösung, in Fig. 1 beispielhaft als ein Calciumbeutel 13 ausgestaltet, eine Calciumlösung in die Leitung 11 abzugeben. Aus der Quelle für Calciumlösung wird beispielsweise eine $CaCl_2$-Lösung zugeführt. Diese kann eine Calciumkonzentration von 91 mmol/l aufweisen.

Der Filter 3 ist auf der Dialysatseite mit einer Dialysatquelle, z. B. einem Dialysatbeutel 15, verbunden. Aus der Quelle für Dialysat wird Dialysat, z. B. ein Citrat-Calcium(kurz: Ci-Ca)-Dialysat K2, dem Filter 3 zugeführt. Der Filter 3 ist ferner verbunden mit einem Ablauf 17.

Die Leitung 11 ist ferner mit einer Quelle für Substituat, hier in Gestalt eines Substituatbeutels 19, verbunden. Der Beutel 19 enthält ein calciumhaltiges Substituat, bei welchem es sich beispielsweise um das Produkt "multiBic" der Firma Fresenius Medical Care Deutschland GmbH, handeln kann. Rein exemplarisch ist der Beutel 19 - als Beispiel einer Quelle für Substituat - angeordnet zur Zugabe des Substituats in Postdilution, also an einer Stelle des Blutkreislaufs 1, welche in Strömungsrichtung des Bluts hinter dem Filter 3 liegt.

Der mittels der Blutpumpe 7 eingestellte Blutfluss kann auf 100 ml/min eingestellt sein. An einer in Fig. 1 nicht gezeigten Pumpe zum Fördern von Citratlösung aus dem Citratbeutel 9 heraus kann ein Fluss von 180 ml/h eingestellt sein. An der Zugabeeinrichtung 12, hier als Calciumpumpe ausgestaltet, zum Fördern von Calciumlösung aus dem Calciumbeutel 13 heraus und in den extrakorporalen Blutkreislauf 1 hinein kann ein Fluss von 45 ml/h eingestellt sein. An einer in Fig. 1 nicht gezeigten Pumpe zum Fördern von Dialysat aus dem Dialysatbeutel 15 heraus kann ein Fluss von 1800 ml/h eingestellt sein. An einer in Fig. 1 nicht gezeigten Pumpe zum Fördern von Substituat aus dem Substituatbeutel 19 heraus kann ein Fluss von 1000 ml/h eingestellt sein. Der Abfluss kann ca. 3100 ml/h betragen. Seine Größe hängt ab von der vom Arzt angestrebten Netto-Ultrafiltrationsrate.

Aus Fig. 1 ist ersichtlich, dass dem Patienten P Calcium jeweils aus dem Calciumbeutel 13, dem Dialysatbeutel 15 sowie aus dem Substituatbeutel 19 zugeführt wird.

Die Zufuhr des Calciums bzw. der Calciumlösung aus dem Calciumbeutel 13 mittels der Calciumpumpe 12 wird mittels einer Steuereinrichtung 20 der Vorrichtung 4 gesteuert oder geregelt. Ein entsprechende Signalverbindung ist als Strich-Punkt-Linie angedeutet.

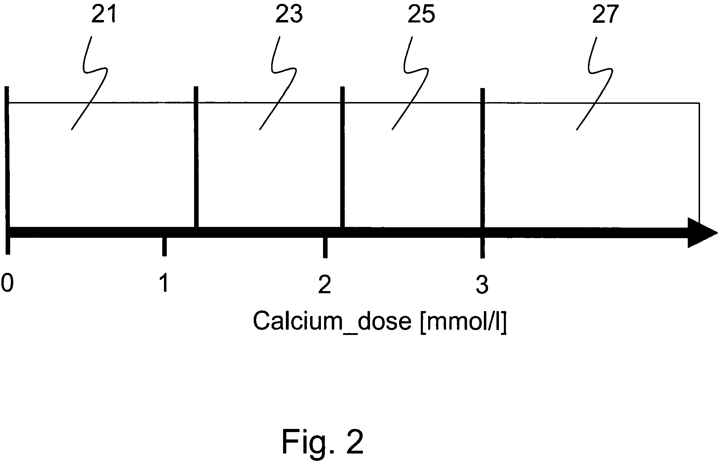

**Fig. 2** zeigt exemplarisch mögliche Einstellbereiche für eine Calciumdosierung zum Zweck des Calciumausgleichs. Fig. 2 zeigt ferner Grenzen des Einstellbereichs. Die Dimension der Dosierung für Calcium, in Fig. 2 als Calcium_dose bezeichnet, beträgt Millimol pro Liter [mmol/l]. Der im Beispiel der Fig. 2 als zulässig erachtete oder zugelassene Einstellbereich für die Dosierung von Calcium zum Calciumausgleich erstreckt sich über den Wertebereich, welcher von 0 bis 3 mmol/l reicht.

Das Festlegen einer Calciumdosis in einem Bereich 21 entspricht der Substitution einer Menge an Calcium, welche geringer ist als die erwartete Menge eines mittels der Blutbehandlung entfernten Calciums. Eine Einstellung in diesem Bereich könnte zu einem unerwünschten Calciumverlust führen, daher kann das Einstellen einer derartigen Dosierung in bestimmten erfindungsgemäßen Ausführungsformen mit dem Anzeigen eines Hinweises, eines optischen oder akustischen Alarms oder dergleichen einhergehen. Alternativ kann der Versuch, eine Calciumdosis im Bereich 21 einzustellen auch ein Zeichen dafür sein, dass weniger Calcium als erwartet aus dem Blut durch die Membran gelangt und mit dem Effluent entfernt wird. Dies kann Folge einer reduzierten Permeabilität der Membran sein. Auf diese Möglichkeit kann der Anwender oder die Behandlungsvorrichtung in bestimmten erfindungsgemäßen Ausführungsformen hingewiesen werden.

Das Einstellen einer Calciumdosis, welche in einem Bereich 23 liegt, entspräche den Erwartungen und würde zu keinem Hinweis an den Benutzer führen.

Das Einstellen einer Calciumdosis, welche in einem Bereich 25 liegt, entspricht einer Calciumsubstitution oberhalb der erwarteten Calciumentfernung über das Effluent. Ein Hinweis an den Benutzer hierüber ist in bestimmten erfindungsgemäßen Ausführungsformen vorgesehen, in anderen unterbleibt ein solcher Hinweis. Hingewiesen werden könnte auch darauf, dass möglicherweise eine Citratakkumuluation vorliegt, da diese oft mit einem erhöhten Calciumsubstitutionsbedarf verbunden ist.

Das Einstellen einer Calciumdosis, welche in einem Bereich 27 liegt, würde als zu hoch angesehen und wäre nicht zulässig. Eine Auswählbarkeit eines solchen, unzulässigen Wertes für einen Calciumausgleich mittels der erfindungsgemäßen Einrichtung oder Pumpe wäre erfindungsgemäß nicht möglich. Der Benutzer kann in manchen erfindungsgemäßen Ausführungsformen, sollte er eine Calciumdosis im Bereich 27 einstellen wollen, den Hinweis erhalten, er möge prüfen, ob unter den gegebenen Umständen nicht eine andere Behandlungsoption als die eingestellte bzw. ausgeführte geeigneter ist und diese ggf. einstellen.

Ferner kann in manchen erfindungsgemäßen Ausführungsformen eine automatische Anpassung anderer Parameter als der Calciumdosis erfolgen, welche zu einer ausgewogenen Calciumbilanz führen können. Eine Liste mit automatisch zulässigen Änderungen kann hinterlegt sein. Sollten Anpassungen erforderlich sein oder gar automatisch durchgeführt werden, so ist in bestimmten Ausführungsformen vorgesehen, den Benutzer hierüber zu informieren.

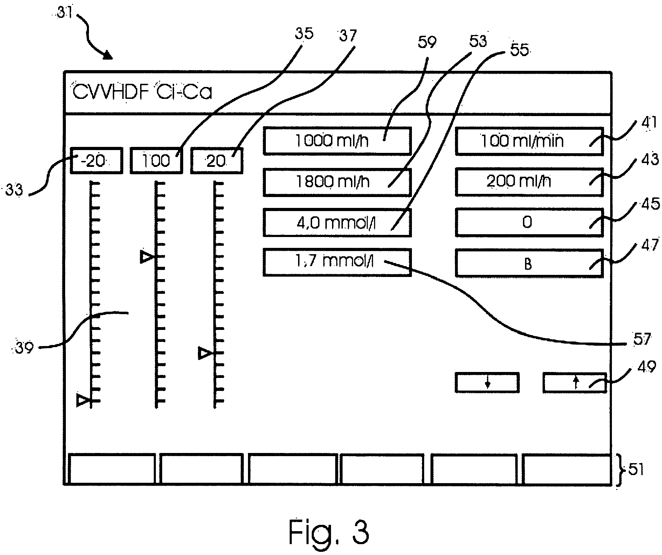

**Fig. 3** zeigt eine erfindungsgemäße Benutzeroberfläche 31 zur Durchführung des erfindungsgemäßen Verfahrens.

Auf ihrer linken Seite sind der Benutzeroberfläche 31 Angaben zum arteriellen Druck (Bezugzeichen 33), zum venösen Druck (Bezugzeichen 35) und zum Transmembrandruck (Bezugzeichen 37), hier jeweils in mmHg angegeben, zu entnehmen. Die entsprechende Werte können dabei sowohl als Zahlenangaben als auch als veränderliche Markierungen auf Skalen 39 abgelesen werden.

Die Benutzeroberfläche 31 weist ferner eine Anzeige 41 des aktuell eingestellten oder gemessenen Blutflusses, eine Anzeige der Ultrafiltrationsrate 43, eine Angabe 45 darüber, ob eine kontinuierliche Heparingabe erfolgt (hier mit 0 für AUS), und eine Angabe 47 darüber, ob eine Heparingabe per Bolus (hier mit B für "Bolus eingestellt") erfolgt.

Die Benutzeroberfläche 31 weist ferner Einstellmöglichkeiten 49 für einen Pegel des venösen Blasenfängers und Steuerinterfaces 51 zum Anzeigen oder Steuern weiterer Behandlungsdaten oder -optionen auf.

Zusätzlich umfasst die Benutzeroberfläche 31 eine Anzeige 53 zum Anzeigen des eingestellten Dialysatflusses (entspricht der Dialysatrate), eine Anzeige 55 zum Anzeigen des eingestellten Citratflusses in Relation zum Blutfluss, und eine Anzeige 57 zum Anzeigen des eingestellten Calciumflusses in Relation zum Filtratfluss. Alle oder einige der vorgenannten und der im Folgenden erwähnten Anzeigen können in bestimmten erfindungsgemäßen Ausführungsformen auch zum Verändern der eingestellten Flussraten dienen. So können sie durch Berühren der entsprechenden Displayflächen einstellbar sein.

Die Benutzeroberfläche 31 unterscheidet sich in dem vorstehend Beschriebenen nicht von einer bereits bekannten Benutzeroberfläche, wie sie auch für eine andere Behandlungsoption wie die CVVHD (Kontinuierliche venovenöse Hämodialyse) in Kombination mit Citrat-Calcium-Antikoagulation verwendet werden kann.

Neu oder hinzugekommen ist hingegen die Anzeige 59 des Substituatflusses. Die Benutzeroberfläche 31 umfasst damit zusätzlich auch jene Angabe, welche für die Durchführung eines als CVVHDF (Kontinuierliche venovenöse Hämodiafiltration) bekannten Verfahrens kombiniert mit Citrat-Calcium-Antikoagulation, wie in der oberen linken Ecke der Benutzeroberfläche 31 angezeigt ist, relevant ist.

Die in Fig. 3 dargestellte Benutzeroberfläche 31 ist damit eine Weiterbildung einer Benutzeroberfläche, wie sie bei der CVVHD verwendet werden kann. Die erfindungsgemäße Benutzeroberfläche 31 unterscheidet sich somit vorteilhaft nur geringfügig von einer Benutzeroberfläche, die der Anwender bereits aus einer anderen Blutbehandlungsoption kennt. Sie kann damit in bestimmten erfindungsgemäßen Ausführungsformen auf einfache Weise ausgehend von einer Anzeige für CVVHD auf eine Anzeige für CVVHDF angepasst werden und umgekehrt. Es kann genügen, bestimmte, wenige Anzeigen und/oder Einstellmöglichkeiten mehr oder weniger anzubieten in Abhängigkeit davon, welche der vorgenannten Behandlungsoptionen verfolgt werden soll. Hiermit verbunden ist der Vorteil, dass der Benutzer eine ihm bereits von der Durchführung einer Behandlungsoption bekannte Benutzeroberfläche in nur geringfügig verändertem Aufbau der Benutzeroberfläche auch beim Durchführen einer weiteren Behandlungsoption oder des erfindungsgemäßen Verfahrens verwenden kann. Ein Irritieren des Benutzers kann damit ebenso vermieden werden wie die Notwendigkeit, dass der Benutzer mit einer Vielzahl von Benutzeroberflächen vertraut sein muss.

Sollte sich bei Einstellen eines gewünschten Wertes für die Calciumzugabe mittels der Calciumpumpe durch den Benutzer eine Unmöglichkeit einer eingestellten Konstellation der diversen Eingabeparameter (Flüsse, Calciumdosis, usw.) ergeben, beispielsweise im Sinne eines an sich negativen Wertes des errechneten Calciumflusses aus der Calciumpumpe, so wird in bestimmten erfindungsgemäßen Ausführungsformen die Calciumdosis auf Null, auf den minimal einstellbaren Wert, auf einen unteren Schwellenwert oder auf einen hiervon abweichenden, vorbestimmten Defaultwert angehoben. Der minimal einstellbare Wert beträgt in einigen erfindungsgemäßen Ausführungsformen einen auf volle 0,1 mmol/l aufgerundeten Wert. Er kann beispielsweise gemäß der oben angegebenen Formel (1) oder (1') oder auf andere geeignete Weise berechnet werden.

Einer derartigen technischen Unmöglichkeit der rechnerisch ermittelten erforderlichen Calciumdosis (also beispielsweise ein negativer Wert) kann beispielsweise auch Rechnung getragen werden, indem sich die gewünschte aber nicht in die Praxis umsetzbare Calciumdosis nicht vom Benutzer eingeben lässt. Beispielsweise kann bereits der Displayrechner, oder jede andere geeignete Einrichtung, entsprechend programmiert sein, um dem Anwender lediglich sinnvolle und/oder technisch mögliche Dosierungen zur Auswahl stellen. Gibt der Benutzer dennoch technisch unmögliche oder zumindest nicht zugelassene Werte für die Calciumdosierung mittels der Calciumpumpe ein, was in bestimmten erfindungsgemäßen Ausführungsformen zunächst möglich sein kann, in anderen hingegen von Anfang an nicht, so kann vorzugsweise ein Hinweis erfolgen, um den Anwender z. B. über die Unmöglichkeit zu informieren.

In bestimmten erfindungsgemäßen Ausführungsformen erfolgt ein Hinweis an der Anwender immer dann, wenn eine maschinenseitig, d. h. automatisch, vorgenommene Einstellung der Calciumdosis von jener abweicht, die der Anwender gewünscht und einzugeben versucht hat. Dies kann dazu beitragen, dass der Anwender das Verhalten des Gerätes nachvollziehen kann.

In einigen erfindungsgemäßen Ausführungsformen kann die Calciumdosis automatisch auf Null, auf einen Mindestwert oder auf den sich rechnerisch ergebenden Wert (z. B. nach Formel (1) oder (1')) gesetzt werden, wenn sich mit oder ohne Zutun des Anwenders andere Einstellgrößen als die Calciumdosis - wie beispielsweise der Dialysatfluss oder der Substituatfluss - ändern, welche Einfluss auf den errechneten Calciumfluss haben. Der Anwender kann hierüber informiert werden durch Hinweis, Alarm, Textfeld, usw.

Eine Ausnahme von dem zuletzt genannten Vorgehen ist in manchen erfindungsgemäßen Ausführungsformen dann vorgesehen, wenn nach Erreichen des Bilanzziels, das heißt eines Ziels für den kumulierten Entzug von Wasser aus dem Blut, ein Stopp oder eine Ende der Ultrafiltration erreicht ist und die Maschine aus diesem Grund in einen veränderten Zustand (bezogen auf Parameterwert, Flüsse und dergleichen) übergeht. Ist dies der Fall, so bedarf es in bestimmten erfindungsgemäßen Ausführungsformen keiner Anpassung der Calciumdosis. Auch in dieser Situation kann der Anwender hierüber informiert werden durch Hinweis, Alarm, Textfeld, usw.

In bestimmten erfindungsgemäßen Ausführungsformen ist vorgesehen, dass vor einer automatischen Änderung von Parameterwerten, z. B. für die Calciumpumpe, geltende Einstellungen für beliebige Einstellgrößen der erfindungsgemäßen Einrichtung oder Vorrichtung (Calciumdosis und andere) automatisch von der erfindungsgemäßen Einrichtung oder Vorrichtung gespeichert werden. Mittels der gespeicherten Werte können - automatisch oder auf Anregung durch den Anwender - die Parameter der Maschine auf die bisherigen Einstellungen zurückgesetzt werden, jedenfalls dann, wenn die bisherigen Einstellungen erneut technisch und medizinisch sinnvoll und möglich und gewünscht sind. Der Anwender kann einen Hinweis erhalten, dass aufgrund bestimmter Änderungen nun frühere Werte erneut eingestellt werden können. Beispiele für derartige Einstellungen und Parameter umfassen, ohne hierauf beschränkt zu sein, die Calciumdosis, den Blutfluss, den Dialysatfluss, den Substituatfluss und andere.

In einigen erfindungsgemäßen Ausführungsformen kann bei Start der Behandlung ein in einer früheren Behandlung gesetzter Merker zunächst als gelöscht initialisiert werden, welcher z. B. stets dann gesetzt wird, wenn die Calciumdosis automatisch angehoben wurde. Der Merker kann gesetzt werden, wenn der Anwender eine Calciumdosis eingibt. Durch das Setzen des Merkers wird der bisher gültige Wert der Calciumdosis zumindest vorübergehend gespeichert. Ist der Merker gesetzt und ist nach Änderung eines anderen Parameters (optional mit Ausnahme des automatischen Absenkens der Ultrafiltrationsrate auf Null bei Erreichen des Bilanzziels) eine Senkung der Calciumdosis möglich oder ein Anheben der Calciumdosis erforderlich oder ratsam, so kann ein Hinweis an den Anwender erfolgen, dass dieser nun die Calciumdosis senken kann. Der Merker kann daraufhin gelöscht werden. Alternativ kann eine solche Anpassung auch automatisch erfolgen.

In einigen erfindungsgemäßen Ausführungsformen ist die Auswahl der Calciumdosis auf eine abzählbar endliche Anzahl von Dosisstufen beschränkt. In diesen Ausführungsformen ist es nicht oder nicht in jedem Fall vorgesehen, die niedrigst mögliche Calciumdosis, wie sie z. B. mittels Formel (1) oder (1') berechnet wurde, einzustellen oder anzusteuern. In diesen Ausgestaltungen können beispielsweise 0,1 mmol/l oder 0,2 mmol/l als Einstellweite oder Einstellschritte vorgesehen sein, was sich vorteilhaft bewährt hat. Daher werden für die verfügbaren Einstellschritte in einigen erfindungsgemäßen Ausführungsformen eine Schrittweite von 0,1 mmol/l oder 0,2 mmol/l vorgesehen. Die Einschränkung der möglichen Anpassung um oder auf jeweils mindestens 0,1 mmol/l oder 0,2 mmol/l erlaubt vorteilhaft ein wenig aufwendiges Vorbereiten der bei der Behandlung zum Einsatz kommenden Hardware, insbesondere der Zugabeeinrichtung, mittels welcher der Calciumausgleich erfolgt, und ihrer Ansteuerung. Zudem erlaubt dies ein Design der Benutzeroberfläche, bei dem die kleinsten Einstellschritte einerseits eine Individualisierung der Behandlung ermöglichen und andererseits durch Verzicht auf eine darüber hinaus gehende Präzision der Anzeige, die bei kleineren Einstellschritten erforderlich wäre, die Darstellung überflüssiger Information vermeidet. Dies dient vorteilhaft der Klarheit der Benutzeroberfläche.

Im Folgenden werden drei Situationen erläutert, welche das erfindungsgemäße Verfahren noch besser verstehen lassen.


**Situation 1**

In einer Ausgangskonfiguration der erfindungsgemäßen Vorrichtung gilt:

| | |
|---|---|
| Blutfluss | 100 ml/min |
| Substituatfluss (Q_Sub) | 1000 ml/h |
| Dialysatfluss (Q_Dia) | 1800 ml/h |
| Citratdosis | 4 mmol/l |
| Calciumdosis | 1,7 mmol/l |

(fortgesetzt)

| | |
|---|---|
| Netto-Ultrafiltrationsrate (Q_UF) | 100 ml/h |

Dabei ergeben sich die intern errechneten Flüsse für die Citratlösung zu 176,47 ml/h und für die Calciumlösung zu 37,95 ml/h.

Situation 1 spiegelt einen normalen Betriebszustand wider.

Verringert der Anwender die Calciumdosis nun auf beispielsweise 1,2 mmol/l, so verändert sich der Calciumfluss zu 22,18 ml/h bei sonst unveränderten Werten. Ein Hinweis an den Anwender mit dem Text "niedrige Calciumdosis" mag angebracht sein - ggf. hat der Filter an Funktion eingebüßt.

Wird die Calciumdosis hingegen auf 0,5 mmol/l verringert, so sinkt der Calciumfluss auf 0,38 ml/h ab bei sonst unveränderten Werten. Ein Hinweis an den Anwender mit dem Text "untere Grenze für Calciumdosis erreicht" mag anzeigt sein.

Wird die Calciumdosis allerdings auf 0,4 mmol/l verringert, so müsste der Calciumfluss rechnerisch auf -2,70 ml/h abgesenkt werden zum Erzielen eines korrekten Calciumausgleichs bei ansonsten unveränderten Werten. Eine solche Einstellung ist aber erkennbar sinnlos und wird daher nicht angeboten oder zugelassen.

**Situation 2**

In einer anderen Ausgangskonfiguration der erfindungsgemäßen Vorrichtung gilt:

| | |
|---|---|
| Blutfluss | 100 ml/min |
| Substituatfluss | 1000 ml/h |
| Dialysatfluss | 1800 ml/h |
| Citratdosis | 4 mmol/l |
| Calciumdosis | 0,7 mmol/l |
| Netto-Ultrafiltrationsrate | 100 ml/h |

Dabei ergeben sich die intern errechneten Flüsse für die Citratlösung zu 176,47 ml/h und für die Calciumlösung zu 6,58 ml/h.

Die Calciumdosis ist dabei niedrig, sie ist aufgrund des hohen Dialysatflusses jedoch noch zulässig. Durch Reduzieren des Dialysatflusses kann allerdings eine Grenze der Calciumdosis erreicht werden, wie im Folgenden zu sehen ist.

Verringert der Anwender den Dialysatfluss nämlich auf beispielsweise 800 ml/h, so verändert sich der errechnete Calciumfluss auf -0,47 ml/h bei ansonsten unveränderten Werten. Ein automatisches Anheben der Calciumdosis auf einen niedrigsten der möglichen Werte ist hier angezeigt, ggf. unter Hinweis an den Anwender.

Nachdem die Calciumdosis automatisch auf z. B. 0,8 mmol/l angehoben wurde, steigt der Calciumfluss auf 1,62 ml/h an. Dies ist ein zulässiger Wert.

**Situation 3**

In einer wiederum anderen Ausgangskonfiguration der erfindungsgemäßen Vorrichtung gilt:

| | |
|---|---|
| Blutfluss | 100 ml/min |
| Substituatfluss | 600 ml/h |
| Dialysatfluss | 1800 ml/h |
| Citratdosis | 4 mmol/l |
| Calciumdosis | 0,4 mmol/l |
| Netto-Ultrafiltrationsrate | 100 ml/h |

Dabei ergeben sich die intern errechneten Flüsse für die Citratlösung zu 176,47 ml/h und für die Calciumlösung zu 1,71 ml/h.

Die Calciumdosis ist dabei niedrig, sie ist aufgrund des niedrigen Substituatflusses jedoch noch zulässig. Durch Erhöhen des Substituatflusses kann allerdings eine Grenze der Calciumdosis erreicht werden, wie im Folgenden zu sehen ist.

Erhöht der Anwender den Substituatfluss beispielsweise auf 1000 ml/h, so verändert sich der zum Erzielen eines Calciumausgleichs als erforderlich errechnete Calciumfluss auf -2,70 ml/h bei ansonsten unveränderten Werten. Ein automatisches Anheben der Calciumdosis auf einen niedrigsten der möglichen Werte ist auch hier angezeigt, ggf. wiederum unter Hinweis an den Anwender.

Nachdem die Calciumdosis automatisch auf z. B. 0,5 mmol/l angehoben wurde, steigt der Calciumfluss auf 0,38 ml/h an bei sonst unveränderten Werten. Dies ist ein zulässiger Wert.

**Bezugszeichenliste**

| 1 | Blutkreislauf |
|---|---|
| 3 | Filter |
| 4 | Vorrichtung zum extrakorporalen Behandeln von Blut |
| 5 | Leitung |
| 7 | Blutpumpe |
| 9 | Citratbeutel |
| 11 | Leitung |
| 12 | Zugabeeinrichtung, hier: Calciumpumpe |
| 13 | Calciumbeutel |
| 15 | Dialysatbeutel |
| 17 | Ablauf |
| 19 | Substituatbeutel |
| 20 | Steuereinrichtung |
| 21 | Bereich |
| 23 | Bereich |
| 25 | Bereich |
| 27 | Bereich |
| 31 | Benutzeroberfläche |
| 33 | Angaben zum arteriellen Druck |
| 35 | Angaben zum venösen Druck |
| 37 | Angaben zum Transmembrandruck |
| 39 | Skalen |
| 41 | Anzeige des aktuell eingestellten oder gemessenen Blutflusses |
| 43 | Anzeige der Ultrafiltrationsrate |
| 45 | Angabe über kontinuierliche Heparingabe |
| 47 | Angabe über eine Heparingabe per Bolus |
| 49 | Einstellmöglichkeiten für einen Pegel des venösen Blasenfängers |
| 51 | Steuerinterfaces zum Anzeigen oder Steuern weiterer Behandlungsdaten oder -optionen |
| 53 | Anzeige des eingestellten Dialysatflusses |
| 55 | Anzeige des eingestellten Citratflusses in Relation zum Blutfluss |
| 57 | Anzeige des eingestellten Calciumflusses in Relation zum Filtratfluss |
| 59 | Anzeige des Substituatflusses |

| Ca-Dosis_min | minimale Calciumdosis oder als kleinstzulässig erachteter unterer Wert des Wertebereichs; |
|---|---|
| [Ca]_Sub | Calciumkonzentration des Substituats; |
| [Ca]_Lsg | Calciumkonzentration der Calciumlösung; |
| Q_Fil | Filtratfluss |
| Q_Sub | Substituatfluss |
| Q_Dia | Dialysatfluss |
| Q_Ca_min | minimaler Calciumfluss |
| Q_Ca | Calciumfluss |
| Q_Ci | Citratfluss |
| Q_UF | Nettofluss der Ultrafiltration |
| Q_Hep | Heparinfluss. |

**Patentansprüche**

1. Verfahren zum Steuern oder Regeln einer Vorrichtung (4) zum extrakorporalen Behandeln von Blut in einem extrakorporalen Blutkreislauf (1) unter Zugabe von Citrat zum Zweck der Antikoagulation, wobei die Vorrichtung (4) eine Zugabeeinrichtung (12) zum Zugeben einer Calciumlösung in den extrakorporalen Blutkreislauf (1) aufweist, oder zum Steuern der Zugabeeinrichtung (12),
mit den Schritten:

   - Ausgeben eines Signals an die Zugabeeinrichtung (12) zum Verändern einer Einstellung der Zugabeeinrichtung (12), wobei die Einstellung einer Zugabe von Calcium, einer Calciumdosierung oder -konzentration oder -menge oder -rate entspricht oder bewirkt;

   **gekennzeichnet durch** die Schritte

   - Festlegen des Signals unter Berücksichtigung eines Calciumgehalts oder einer Calciumkonzentration einer beim extrakorporalen Behandeln des Bluts verwendeten Substituatlösung; und
   - Automatisches Ermitteln, ohne Zutun eines Benutzers, des Wertebereichs eines zulässigen oder zugelassenen Einstellbereichs für das mittels der Zugabeeinrichtung (12) in den extrakorporalen Blutkreislauf (1) eingebrachte oder einzubringende Calcium, insbesondere dessen Menge, Konzentration oder Dosierung.

2. Verfahren nach Anspruch 1, mit dem Schritt:

   - Festsetzen eines als kleinstzulässig erachteten unteren Wertes des Einstellbereichs, insbesondere auf einen Wert gleich oder größer Null.

3. Verfahren nach Anspruch 1 oder 2, mit dem Schritt:

   - Erkennen eines an der Vorrichtung (4) zum extrakorporalen Behandeln von Blut eingestellten oder ablaufenden Blutbehandlungsmodus.

4. Verfahren nach einem der vorangegangenen Ansprüche, mit dem Schritt:

   - Ermitteln des als kleinstzulässig erachteten unteren Schwellenwerts des Wertebereichs des Einstellbereichs nach der Formel

$$Ca\_Dosis\_\min = \frac{[Ca]\_Sub * Q\_Sub + [Ca]\_Lsg * Q\_Ca\_\min}{Q\_Fil + Q\_Ca\_\min}$$

   oder nach der Formel

$$Ca\_Dosis\_\min = \frac{[Ca]\_Sub * Q\_Sub}{Q\_Fil}$$

   wobei Q_Fil die Summe von wenigstens zwei Flüssen aus der Gruppe ist, welche wenigstens Q_Sub, Q_Dia, Q_Ci, Q_Hep, und Q_UF aufweist oder hieraus besteht,
   wobei gilt:

   | | |
   |---|---|
   | Ca-Dosis_min | minimale Calciumdosis oder als kleinstzulässig erachteter unterer Wert des Wertebereichs; |
   | [Ca]_Sub | Calciumkonzentration des Substituats; |
   | [Ca]_Lsg | Calciumkonzentration der Calciumlösung; |
   | Q_Fil | Filtratfluss; |
   | Q_Sub | Substituatfluss; |
   | Q_Dia | Dialysatfluss; |
   | Q_Ca_min | minimaler Calciumfluss; |

(fortgesetzt)

| Q_Ca | Calciumfluss; |
|---|---|
| Q_Ci | Citratfluss; |
| Q_Hep | Heparinfluss; und |
| Q_UF | Nettofluss der Ultrafiltration. |

5. Verfahren nach einem der vorangegangenen Ansprüche, mit dem Schritt:

- Erlauben eines Einstellens der Calciumdosierung, - menge oder -konzentration durch den Benutzer der Vorrichtung (4) oder der Zugabeeinrichtung (12) nur im Wertebereich des Einstellbereichs und/oder nicht unterhalb des als kleinstzulässig erachteten unteren Wertes.

6. Verfahren nach einem der vorangegangenen Ansprüche, mit dem Schritt:

- Ausgeben eines Hinweises bei erfolgtem oder erfolgendem Wechsel eines Behältnisses (19) für das verwendete Substituat oder bei Veränderung der Zusammensetzung oder des bzw. der Calciumgehalts oder -konzentration des verwendeten Substituats.

7. Verfahren nach einem der vorangegangenen Ansprüche, mit dem Schritt:

- Anpassen des als kleinstzulässig erachteten unteren Schwellenwerts des Wertebereichs aufgrund einer Veränderung der Förderleistung von Blutpumpe (7) und/oder Dialysatpumpe und/oder einer anderen Pumpe der Vorrichtung (4).

8. Verfahren nach einem der vorangegangenen Ansprüche, mit dem Schritt:

- Zulassen eines Absenkens der mittels der Zugabeeinrichtung zugegebenen Calcium-Dosis unter den zuvor als kleinstzulässig erachteten unteren Wert des Einstellbereichs durch den Benutzer, wenn nach Änderung des Blutflusses die sich aufgrund der Änderung ergebende minimale Calciumdosis niedriger liegt als vor der Änderung.

9. Steuereinrichtung (20), programmiert zum Ausführen des Verfahrens nach wenigstens einem der Ansprüche 1 bis 8.

10. Benutzeroberfläche (31), welche eine Steuereinrichtung (20) gemäß Anspruch 9 aufweist oder funktionell mit einer solchen verbunden ist, mit einer Eingabeeinrichtung (51) zum Eingeben einer Calciumdosierung oder -menge oder -konzentration, deren Abgabe mittels der Steuereinrichtung (20) bewirkt werden soll.

11. Benutzeroberfläche (31) nach Anspruch 10, aufweisend eine Anzeige, vorgesehen um den als kleinstzulässig erachteten unteren Wert anzuzeigen.

12. Einrichtung (12) zum Zugeben einer Calciumlösung in den extrakorporalen Blutkreislauf, programmiert zum Durchführen eines, insbesondere automatisierten, Verfahrens nach wenigstens einem der Ansprüche 1 bis 8.

13. Vorrichtung (4) zum extrakorporalen Behandeln von Blut, welche zum Durchführen des Verfahrens gemäß einem der Ansprüche 1 bis 8 ausgestaltet ist und/oder wenigstens eine Steuereinrichtung (20) gemäß Anspruch 9 und/oder eine Benutzeroberfläche (31) gemäß Anspruch 10 oder 11 und/oder eine Einrichtung (12) zum Zugeben einer Calciumlösung in den extrakorporalen Blutkreislauf gemäß Anspruch 12 aufweist.

14. Vorrichtung (4) nach Anspruch 13, ausgestaltet als Dialysevorrichtung, insbesondere als Hämodiafiltrationsvorrichtung oder Hämofiltrationsvorrichtung.

**Claims**

1. A method for controlling or regulating an apparatus (4) for extracorporeally treating blood in an extracorporeal blood circuit (1) while adding citrate for the purpose of anticoagulation, wherein the apparatus (4) comprises a dispensing device (12) for dispensing a calcium solution into the extracorporeal blood circuit (1), or for controlling the dispensing device (12),
with the steps:

- outputting a signal to the dispensing device (12) for altering a setting of the dispensing device (12), wherein the setting corresponds to or effects an addition of calcium, a calcium dosage or concentration or amount or rate;

**characterized by** the steps

- defining the signal with regard to a calcium content or a calcium concentration of a substituate solution used in extracorporeally treating the blood; and
- automatically detecting the range of permissible or allowed settings for the calcium introduced or to be introduced into the extracorporeal blood circuit (1) by means of the dispensing device (12), particularly its amount, concentration or dosage, without a user being involved.

2. The method according to claim 1, with the step:

- determining a value regarded as the lowest permissible value of the setting range, particularly to a value equal to or higher than zero.

3. The method according to claim 1 or 2, with the step:

- recognizing a blood treatment mode set or running at or in the apparatus (4) for extracorporeally treating blood.

4. The method according to one of the preceding claims, with the step:

- detecting the lower threshold value regarded as the lowest permissible value of the range of settings according to the formula

$$Ca\_Dosis\_\min = \frac{[Ca]\_Sub * Q\_Sub + [Ca]\_Lsg * Q\_Ca\_\min}{Q\_Fil + Q\_Ca\_\min}$$

or according to the formula

$$Ca\_Dosis\_\min = \frac{[Ca]\_Sub * Q\_Sub}{Q\_Fil}$$

wherein Q_Fil is the sum of at least two flows from the group that comprises at least or consists of Q_Sub, Q_Dia, Q_Ci, Q_Hep, and Q_UF,
wherein applies:

| | |
|---|---|
| Ca-Dosis_min | minimum calcium dose or lower value of the values range regarded as the lowest permissible value; |
| [Ca]_Sub | calcium concentration of the substituate; |
| [Ca]_Lsg | calcium concentration of the calcium solution; |
| Q_Fil | filtrate flow; |
| Q_Sub | substituate flow; |
| Q_Dia | dialysate flow; |
| Q_Ca_min | minimum calcium flow; |
| Q_Ca | calcium flow; |
| Q_Ci | citrate flow; |
| Q_Hep | heparin flow; and |
| Q_UF | net flow of the ultrafiltration. |

**5.** The method according to one of the preceding claims, with the step:

- allowing setting a calcium dosage, amount or concentration by the user of the apparatus (4) or the dispensing device (12) only in the range of values of the setting range and/or not below the lower value regarded as the lowest permissible value.

**6.** The method according to one of the preceding claims, with the step:

- outputting a message after having exchanged or when exchanging the receptacle (19) for the utilized substitute or when changing the composition or the calcium content or calcium concentration of the utilized substitute.

**7.** The method according to one of the preceding claims, with the step:

- adjusting the lower threshold value of the range of values regarded as the lowest permissible value due to a change in the flow rate of the blood pump (7) and/or the dialysate pump and/or a different pump of the apparatus (4).

**8.** The method according to one of the preceding claims, with the step:

- permitting decreasing the calcium dose added by means of the dispensing device below the lower value formerly regarded as the lowest permissible value of the setting range by the user, if, after changing the blood flow, the minimum calcium dose that results from changing is lower than before changing.

**9.** A control device (20), programmed for executing the method according to at least one of the claims 1 to 8.

**10.** A user interface (31), which comprises a control device (20) according to claim 9 or is functionally connected with such control device (20), with an input device (51) for inputting a calcium dosage or amount or concentration, the addition of which is to be effected by means of the control device (20).

**11.** The user interface (31) according to claim 10, comprising a display, provided for displaying the lower value regarded as the lowest permissible value.

**12.** A device (12) for dispensing a calcium solution into the extracorporeal blood circuit, programmed for executing, particularly automatically, a method according to at least one of the claims 1 to 8.

**13.** An apparatus (4) for extracorporeally treating blood which is embodied for executing the method according to one of the claims 1 to 8 and/or which comprises at least one control device (20) according to claim 9 and/or a user interface (31) according to claim 10 or 11 and/or a device (12) for dispensing a calcium solution into the extracorporeal blood circuit according to claim 12.

**14.** The apparatus (4) according to claim 13, embodied as dialysis apparatus, in particular as hemodiafiltration apparatus or hemofiltration apparatus.


**Revendications**

**1.** Procédé de commande ou de régulation d'un appareil (4) de traitement extracorporel du sang dans un circuit (1) extracorporel de circulation du sang avec addition de citrate pour l'anticoagulation, où l'appareil (4) présente un dispositif d'ajout (12) pour ajouter une solution de calcium dans le circuit extracorporel de circulation du sang, ou de commande du dispositif d'ajout (12),
comprenant les étapes:

- émettre un signal au dispositif d'ajout (12) destiné à modifier un réglage du dispositif d'ajout (12), où le réglage correspond à ou induit une addition de calcium, un réglage de calcium, une concentration de calcium, une quantité de calcium ou un taux de calcium;

**caractérisé par** les étapes

EP 2 670 454 B1

- définir le signal en tenant compte de la teneur ou de la concentration en calcium d'une solution de substitution utilisée lors du traitement extracorporel du sang; et
- déterminer, de façon automatique et sans participation d'un utilisateur, une gamme de valeurs d'un intervalle de réglage autorisé ou admissible pour le calcium introduit ou à introduire au moyen du dispositif d'ajout (12) dans le circuit (1) extracorporel de circulation du sang, en particulier sa concentration, sa quantité ou son dosage.

2. Procédé selon la revendication 1, avec l'étape:

- définir une valeur en tant que valeur de l'intervalle de réglage considérée comme la plus basse admissible, en particulier d'une valeur égale ou supérieure à zéro.

3. Procédé selon la revendication 1 ou 2, avec l'étape:

- reconnaître un mode de traitement du sang réglé ou en cours d'exécution sur l'appareil (4) de traitement extracorporel du sang.

4. Procédé selon l'une des revendications précédentes, avec l'étape:

- déterminer la valeur seuil inférieure considérée comme la plus basse admissible de la gamme de valeurs de l'intervalle de réglage selon la formule

$$Ca\_Dosis\_\min = \frac{[Ca]\_Sub * Q\_Sub + [Ca]\_Lsg * Q\_Ca\_\min}{Q\_Fil + Q\_Ca\_\min}$$

ou selon la formule

$$Ca\_Dosis\_\min = \frac{[Ca]\_Sub * Q\_Sub}{Q\_Fil}$$

où Q_Fil est la somme d'au moins deux flux d'un groupe comprenant Q_Sub, Q_Dia, Q_Ci, Q_Hep et Q_UF, ou étant constitué de ceux-ci,
avec

| | |
|---|---|
| Ca-Dosis_min | dose de calcium minimale ou valeur inférieure de l'intervalle de réglage considérée comme la plus basse admissible; |
| [Ca]_Sub | concentration de calcium du liquide de substitution; |
| [Ca]_Lsg | concentration de calcium de la solution de calcium; |
| Q_Fil | flux de filtrat; |
| Q_Sub | flux de liquide de substitution; |
| Q_Dia | flux de dialysat; |
| Q_Ca_min | flux minimum de calcium; |
| Q_Ca | flux de calcium; |
| Q_Ci | flux de citrate; |
| Q_Hep | flux d'héparine; et |
| Q_UF | flux net de l'ultrafiltration. |

5. Procédé selon l'une des revendications précédentes, avec l'étape:

- autoriser un réglage du dosage de calcium, de la quantité de calcium ou de la concentration de calcium à l'utilisateur de l'appareil (4) ou du dispositif d'ajout (12) uniquement dans la gamme de valeurs de l'intervalle de réglage et/ou pas en dessous de la valeur inférieure considérée comme la plus basse admissible.

6. Procédé selon l'une des revendications précédentes, avec l'étape:

17

- délivrer une indication lors ou après l'échange d'un contenant (19) pour le substitut utilisé ou lors du changement de la composition ou de la teneur ou concentration en calcium, respectivement, du substitut utilisé.

7. Procédé selon l'une des revendications précédentes, avec l'étape:

- réajuster la valeur seuil inférieure de la gamme de valeurs considérée comme la plus basse admissible en raison d'un changement de la capacité de débit d'une pompe à sang (7) et/ou d'une pompe de dialysat et/ou d'une autre pompe de l'appareil (4).

8. Procédé selon l'une des revendications précédentes, avec l'étape:

- permettre à l'utilisateur d'abaisser la dose de calcium ajoutée au moyen du dispositif d'ajout en deçà de la valeur de l'intervalle de réglage considérée préalablement comme la plus basse admissible lorsque, après un changement du flux de sang, la dose de calcium minimale résultant du changement est plus basse qu'avant le changement.

9. Dispositif de commande (20), programmé pour exécuter un procédé selon au moins l'une des revendications 1 à 8 précédentes.

10. Interface utilisateur (31), présentant un dispositif de commande (20) selon la revendication 9 ou étant connectée de façon fonctionnelle avec un tel dispositif, avec un dispositif d'entrée (51) pour entrer une dose de calcium ou une quantité de calcium ou une concentration de calcium dont la distribution doit être induite par le dispositif de commande (20).

11. Interface utilisateur (31) selon la revendication 10 présentant un affichage prévu pour afficher la valeur inférieure considérée comme la plus basse admissible.

12. Dispositif d'ajout (12) pour ajouter une solution de calcium dans le circuit de circulation du sang extracorporel, programmé pour exécuter, en particulier de façon automatique, un procédé selon l'une au moins des revendications 1 à 8.

13. Appareil (4) de traitement extracorporel du sang constitué de façon à pouvoir exécuter le procédé selon l'une des revendications 1 à 8 et/ou présentant au moins un dispositif de commande (20) selon la revendication 9 et/ou une interface utilisateur (31) selon la revendication 10 ou 11 et/ou un dispositif (12) pour ajouter une solution de calcium dans le circuit de circulation de sang extracorporel selon la revendication 12.

14. Appareil (4) selon la revendication 13, formé en tant qu'appareil de dialyse, en particulier en tant qu'appareil d'hémodiafiltration ou d'hémofiltration.

Fig. 1

Fig. 2

Fig. 3

EP 2 670 454 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070066928 A1
- WO 2010029401 A2
- US 20050085760 A1